# EUROPÄISCHE PATENTSCHRIFT

(11) **EP 1 153 127 B1**
(45) Veröffentlichungstag und Bekanntmachung des Hinweises auf die Patenterteilung: **26.07.2006**
(21) Anmeldenummer: 00909211.5
(22) Anmeldetag: 18.02.2000
(51) Int. Cl.: C12N 15/10, C12P 19/34

(54) **VERFAHREN ZUR HERSTELLUNG VON POLYMEREN**
METHOD FOR PRODUCING POLYMERS
PROCEDE DE PRODUCTION DE POLYMERES

(30) Priorität: 19.02.1999 DE 19907080; 24.06.1999 DE 19928843; 27.08.1999 DE 19940752; 27.08.1999 WO PCT/EP99/06316; 26.11.1999 DE 19957116
(43) Veröffentlichungstag der Anmeldung: 14.11.2001
(73) Patentinhaber: Febit Biotech GmbH, 69120 Heidelberg (DE)
(72) Erfinder: STÄHLER, Peer, F., D-69167 Mannheim (DE); STÄHLER, Cord, F., D-69469 Weinheim (DE); MÜLLER, Manfred, D-69198 Schriesheim (DE)
(74) Vertreter: Weiss, Wolfgang
(86) Internationale Anmeldenummer: PCT/EP2000/001356
(87) Internationale Veröffentlichungsnummer: WO 2000/049142

(56) Entgegenhaltungen:
- EP-A- 0 022 242
- EP-A- 0 130 166
- EP-A- 0 316 018
- EP-A- 0 385 410
- WO-A-00/13017
- WO-A-90/00626
- WO-A-94/12632
- WO-A-95/17413
- WO-A-99/14318
- S RAYNER ET AL: "MerMade: An oligodeoxyribonucleotide synthesizer for high throughput oligonucleotide production in dual 96-well plates" PCR METHODS AND APPLICATIONS,US,COLD SPRING HARBOR, NY, Bd. 8, Nr. 7, 1. Juli 1998 (1998-07-01), Seiten 741-747, XP002089330 ISSN: 1054-9803
- L E SINDELAR AND J M JAKLEVIC: "High-throughput DNA synthesis in a multichannel format" NUCLEIC ACIDS RESEARCH,GB,OXFORD UNIVERSITY PRESS, SURREY, Bd. 23, Nr. 6, 1. Januar 1995 (1995-01-01), Seiten 982-987, XP002089329 ISSN: 0305-1048
- LASHKARI D A ET AL: "AN AUTOMATED MULTIPLEX OLIGONUCLEOTIDE SYNTHESIZER: DEVELOPMENT OF HIGH-THROUGHPUT, LOW-COST DNA SYNTHESIS" PROCEEDINGS OF THE NATIONAL ACADEMY OF SCIENCES OF USA,US,NATIONAL ACADEMY OF SCIENCE. WASHINGTON, Bd. 92, Nr. 17, 15. August 1995 (1995-08-15), Seiten 7912-7915, XP000611248 ISSN: 0027-8424

## Beschreibung

Die Erfindung betrifft ein Verfahren zur Herstellung von Polymeren, insbesondere von synthetischen Nukleinsäuredoppelsträngen wahlfreier Sequenz.

### Technischer Hintergrund der Erfindung

Die Manipulation und Konstruktion von genetischen Elementen, wie z.B. Genfragmenten, ganzen Genen oder regulatorischen Bereichen, durch die Entwicklung der DNA-Rekombinationstechnik, welche häufig auch als Gentechnik bezeichnet wird, führte zu einem besonderen Bedarf an gentechnischen Methoden und deren Weiterentwicklung in den Bereichen der Gentherapie, molekularen Medizin (Grundlagenforschung, Vektorentwicklung, Vakzine, Regeneration etc.). Wichtige Anwendungsgebiete sind auch die Wirkstoff-Entwicklung, Wirkstoff-Produktion im Rahmen der Pharmaka-Entwicklung, kombinatorische Biosynthese (Antikörper, Effektoren wie Wachstumsfaktoren, Neurotransmitter etc.), Biotechnologie (z.B. Enzymdesign, Pharming, biologische Herstellungsverfahren, Bioreaktoren etc.), Diagnostika (BioChips, Rezeptoren / Antikörper, Enzymdesign etc.) und Umwelttechnik (spezialisierteodermaßgeschneiderte Mikroorganismen, Produktionsverfahren, Sanierung, Sensoren etc.).

### Stand der Technik

Zahlreiche Methoden, allen voran die enzymgestützten Verfahren, erlauben die gezielte Manipulation von DNA für unterschiedliche Zwecke.

Diese Verfahren müssen allesamt auf vorhandenes genetisches Material zurückgreifen. Dieses ist zwar einerseits weitgehend definiert, erlaubt aber andererseits in einer Art "Baukastensystem" nur eine beschränkte Menge möglicher Kombinationen der jeweiligen vorhandenen und leicht modifizierten Elemente.

Vollsynthetische DNA spielt dabei bisher nur eine untergeordnete Rolle in Form eines dieser kombinatorischen Elemente, mit deren Hilfe gezielte Veränderungen des zur Verfügung stehenden genetischen Materials möglich sind.

Den bekannten Verfahren gemeinsam ist der hohe Arbeitsaufwand, verbunden mit einer gewissen Dauer entsprechender Arbeiten, da die Stufen molekularbiologischer und insbesondere gentechnischer Experimente, wie der Isolierung von DNA, Manipulation, Transfer in geeignete Zielzellen, Vermehrung, erneute Isolation etc., zumeist mehrfach durchlaufen werden müssen. Viele der anfallenden Arbeitsschritte lassen sich nur ungenügend automatisieren und beschleunigen, so daß die entsprechenden Arbeiten zeitaufwendig und arbeitsintensiv bleiben. Bei der Isolation von Genen, die einer funktionellen Untersuchung und einer Charakterisierung des Genproduktes vorausgehen muß, läuft der Informationsfluß in den meisten Fällen von isolierter RNA (mRNA) über cDNA und entsprechende Genbibliotheken über aufwendige Screening-Verfahren zu einem einzelnen Klon. Dieser enthält die gesuchte und in ihm klonierte DNA häufig unvollständig, so daß sich weitere Screening-Verfahren etc. anschließen.

Schließlich ist die oben beschriebene Rekombination von DNA-Fragmenten nur begrenzt flexibel und erlaubt gemeinsam mit dem beschriebenen Arbeitsaufwand nur wenige Optimierungsschleifen. Gerade solche Optimierungen sind angesichts der Vielfalt und Komplexität in der Gerietik, der funktionellen Genomik und der Proteomik, also dem Studium der Wirkungen der Genprodukte, ein Nadelöhr für die weitere Entwicklung der modernen Biologie.

Ein gängiges Verfahren ist die Rekombination durch enzymatische Methoden (in vitro): Hierbei werden DNA-Elemente (isolierte genomische DNA, Plasmide, Amplicons, virale oder bakterielle Genome, Vektoren) zunächst mit entsprechenden Restriktionsenzymen zu Fragmenten mit definierten Enden zerschnitten. Je nach Beschaffenheit dieser Enden können die entstandenen Fragmente neu kombiniert und (ebenfalls enzymatisch) zu größeren DNA-Elementen verbunden werden. Zum Zwecke der Vermehrung der DNA erfolgt dies häufig in einem als Klonierungs-Vektor funktionierenden Plasmid.

Die rekombinierte DNA muss in aller Regel in geeigneten Organismen klonal vermehrt werden (Klonierung) und steht nach diesem zeitaufwendigen Schritt und der Isolierung durch entsprechende Methoden erneut für Manipulationen, wie z.B. Rekombination, zur Verfügung. Limitierend sind bei dieser Methode aber die Schnittstellen der Restriktionsenzyme: Jedes Enzym erkennt auf der (doppelsträngigen) DNA eine spezifische Sequenz, wobei deren Länge in Abhängigkeit vom jeweiligen Enzym zwischen drei und zwölf Nukleotid-Basen beträgt und sich demnach auf jedem DNA-Element nach einer statistischen Verteilung eine bestimmte Anzahl von Schnittstellen befindet, an denen der DNA-Strang durchtrennt wird. Wichtig für die Rekombination ist dabei die Zerlegung der bearbeiteten DNA in definierte Fragmente, die sich anschließend zu der gewünschten Sequenz zusammensetzen lassen. Bis zu einem Grenzbereich der zu schneidenden DNA von 10 - 30 Kilobasenpaaren (kbp) stehen der Rekombinationstechnik ausreichend unterschiedliche und spezifische Enzyme zur Verfügung. Durch Vorarbeiten und kommerzielle Anbieter gibt es außerdem die entsprechenden Vektoren, die die rekombinierte DNA aufnehmen und eine Klonierung (und damit Vermehrung und Selektion) erlauben. Solche Vektoren enthalten angepaßte Schnittstellen für effiziente Rekombination und Integration.

Aus den Regeln der Statistik erwächst aber mit zunehmender Länge der manipulierten DNA das Problem mehrfacher und unerwünschter Schnittstellen. Bei einer Enzym-Erkennungssequenz von 6 Nukleotid-Basen gibt es im statistischen Mittel pro 4000 Basen-Paaren eine Schnittstelle (4⁶) und bei 8 Nukleotid-Basen pro 65.000 (4⁸). Für die Manipulation größerer DNA-Elemente (z.B. virale Genome, Chromosomen etc.) ist die Rekombination mit Restriktionsenzymen daher wenig geeignet.

Bekannt ist außerdem eine Rekombination durch homologe Rekombination in Zellen: Hierbei können größere DNA-Elemente bei Vorliegen von Abschnitten gleicher Sequenz auf den zu rekombinierenden Elementen über den natürlichen Vorgang der homologen Rekombination neu zusammengestellt und manipuliert werden. Diese Rekombinationsereignisse sind wesentlich indirekter als bei der Restriktionsenzym-Methodik und darüber hinaus schwieriger zu steuern. Sie liefern oft deutlich schlechtere Ausbeuten als die oben beschriebene Rekombination mit Restriktionsenzymen.

Ein zweiter wesentlicher Nachteil ist die Beschränkung auf die erwähnten Abschnitte gleicher Sequenz, die damit einerseits überhaupt erst vorliegen müssen und andererseits sehr spezifisch für das jeweilige System sind. Die gezielte Einführung entsprechender Sequenzen bereitet dann ihrerseits erhebliche Schwierigkeiten.

Darüber hinaus bekannt ist die Polymerase Kettenreaktion (Polymerase Chain Reaction: PCR), die eine enzymatische Synthese von DNA (inklusive starker Vervielfältigung) erlaubt, indem die begrenzenden Regionen des zu vervielfältigenden Abschnittes durch kurze, vollsynthetische DNA-Oligomere (sog. Primer) den Start einer DNA-Replikation vorgeben. Dafür müssen diese flankierenden Bereiche allerdings bekannt und für die dazwischenliegende Region spezifisch sein. Die Polymerasen bauen bei Replikation des Stranges allerdings in einer vom jeweiligen Enzym abhängigen Häufigkeit auch falsche Nukleotide ein, so daß stets die Gefahr einer gewissen Verfälschung der Ausgangssequenz besteht. Bei manchen Anwendungen ist diese schleichende Verfälschung sehr störend. In die Primer können bei der chemischen Synthese Sequenzen, wie z.B. die oben beschriebenen Restriktions-Schnittstellen, eingefügt werden. Dies erlaubt eine (begrenzte) Manipulation der gesamten Sequenz. Der vervielfältigte Bereich kann mittlerweile bis in den Bereich von ca. 30 Kbp gehen, allerdings ist der größte Teil dieses DNA-Moleküles die Kopie einer vorher vorhandenen DNA.

Die Primer werden mit automatisierter Festphasen-Synthese hergestellt und sind breit verfügbar, allerdings bedingt die Konfiguration aller bisherigen Synthese-Automaten die Herstellung von zu großen Mengen an Primer-DNA (µmol-Ansätze), wie sie für die PCR nicht notwendig sind, während die Variantenvielfalt beschränkt bleibt.

### Synthetische DNA-Elemente

Seit den Pionierarbeiten von Khorana (u.a. in: Shabarova: Advanced Organic Chemistry of Nucleic Acids, VCH Weinheim;) in den 60er Jahren wurden wiederholt Ansätze beschrieben, um aus chemisch synthetisierten DNA-Molekülen doppelsträngige DNA mit genetischen bzw. kodierenden Sequenzen zusammenzusetzten. Stand der Technik sind hierbei genetische Elemente einer Länge bis ca. 2 kbp, die aus Nukleinsäuren aufgebaut werden. Die chemische Festphasensynthese von Nukleinsäuren und Peptiden ist automatisiert worden. Entsprechende Verfahren und Geräte sind z.B. in der US 4353989 und der US 5112575 beschrieben.

Der Aufbau von doppelsträngiger DNA aus kurzen Oligonukleotiden geschieht nach zwei Verfahren (siehe Holowachuk et. al., PCR Methods and Applications, Cold Spring Harbor Laboratory Press): Einmal erfolgt die Synthese des gesamten Doppelstranges durch Synthese von einzelsträngigen Nukleinsäuren (geeigneter Sequenz), Aneinanderlagerung durch Hybridisieren komplementärer Bereiche und Verbinden des molekularen Rückrates durch z.B. Ligase. Demgegenüber gibt es auch die Möglichkeit einer Synthese von an den Rändern überlappenden Bereichen als einzelsträngige Nukleinsäuren, Aneinanderlagerung durch Hybridisieren, Auffüllen der einzelsträngigen Bereiche durch Enzyme (Polymerasen) und Verbinden des Rückgrates.

Bei beiden Methoden ist die Gesamtlänge des genetischen Elementes durch den Aufwand und die Herstellungskosten für Nukleinsäuren in makroskopischer Säulen-Synthese einerseits und durch die Logistik von getrennt in makroskopischer Säulen-Synthese hergestellten und dann zusammengeführten Nukleinsäuren andererseits auf nur wenige tausend Nukleotid-Basen beschränkt. Damit wird der gleiche Größenbereich abgedeckt wie mit der DNA-Rekombinationstechnik.

Zusammenfassend kann der Stand der Technik als eine Vorgehensweise beschrieben werden, bei der in Analogie zu logischen Operationen das Vorhandene (in diesem Fall genetisches Material in Form von Nukleinsäuren) untersucht und kombiniert wird (Rekombination). Das Ergebnis solcher Rekombinations-Experimente wird dann untersucht und erlaubt Rückschlüsse, u.a. auf die eingesetzten Elemente und ihre kombinierte Wirkung. Die Vorgehensweise kann daher als (punktuell-)analytisch und kombinatorisch beschrieben werden.

Der Stand der Technik erlaubt somit keine systematischen Untersuchungen aller beliebigen Kombinationen. Die Veränderung der kombinierten Elemente ist fast nicht möglich. Das systematische Austesten von Veränderungen ist nicht möglich.

### Gegenstand der Erfindung und damit gelöste Aufgabe

Bereitgestellt werden soll ein Verfahren zur direkten Umsetzung von digitaler genetischer Information (Zielsequenz, Datenbanken etc.) in biochemische genetische Information (Nukleinsäuren), ohne auf die Verwendung von bereits vorhandenen Nukleinsäurefragmenten zurückzugreifen.

Gegenstand der Erfindung ist daher ein Verfahren zur Herstellung von Polymeren, wobei eine Vielzahl von Oligomerbaublöcken auf jeweils unterschiedlichen Bereichen eines gemeinsamen Trägers durch parallele Syntheseschritte aufgebaut, vom Träger abgelöst und untereinander zum Aufbau des Polymeren in Kontakt gebracht werden. Vorzugsweise werden doppelsträngige Nukleinsäure-Polymere mit einer Länge von mindestens 300 bp, insbesondere mindestens 1 000 bp synthetisiert. Die Nukleinsäure-Polymere sind vorzugsweise ausgewählt aus Genen, Genclustern, Chromosomen, viralen und bakteriellen Genomen oder Abschnitten davon. Die zum Aufbau des Polymeren verwendeten Oligomerbaublöcke haben eine Länge von vorzugsweise 5-150, besonders bevorzugt 5-30 Monomereinheiten. In aufeinander folgenden Schritten können jeweils partiell komplementäre Oligonukleotidbaublöcke vom Träger abgelöst werden und unter Hybridisierungsbedingungen miteinander bzw. mit dem Polymer-Zwischenprodukt in Kontakt gebracht werden. Weitere Beispiele für geeignete Polymere sind Nukleinsäureanaloga und Proteine.

In einer besonders bevorzugten Ausführungsform betrifft die Erfindung ein Verfahren zur Herstellung von synthetischer DNA mit beliebig wählbarer Sequenz und damit beliebigen bekannten oder neuen funktionellen genetischen Elementen, die in dieser Sequenz enthalten sind. Dieses Verfahren umfaßt die Schritte
(a) Bereitstellen eines Trägers mit einer Oberfläche, die eine Vielzahl von individuellen Reaktionsbereichen enthält,
(b) ortsaufgelöstes Synthetisieren von Nukleinsäurefragmenten mit jeweils unterschiedlicher Basensequenz an mehreren der individuellen Reaktionsbereiche, und
(c) Ablösen der Nukleinsäurefragmente von individuellen Reaktionsbereichen.

Vorzugsweise werden die Basensequenzen der an individuellen Reaktionsbereichen synthetisierten Nukleinsäurefragmente so gewählt, daß sie sich zu einem Nukleinsäuredoppelstrang-Hybrid zusammenlagern können. Das Ablösen der Nukleinsäurefragmente in Schritt (c) kann dann in einem oder mehreren Schritten unter solchen Bedingungen erfolgen, daß sich mehrere, d.h. zumindest einige der abgelösten Nukleinsäurefragmente zu einem Nukleinsäuredoppelstrang-Hybrid zusammenlagern. Anschließend können die einen Strang des Nukleinsäuredoppelstrang-Hybrids bildenden Nukleinsäurefragmente zumindest teilweise kovalent miteinanderverbunden werden. Dies kann durch enzymatische Behandlung, z.B. mit Ligase, oder/und Auffüllen von Lücken in den Strängen mit DNA-Polymerase erfolgen.

Das Verfahren beinhaltet im Rahmen eines modularen Systems die Synthese sehr vieler einzelner Nukleinsäurestränge, die als Bausteine dienen, wodurch in einem z.B. mikrofluidischen Reaktionsträger eine doppelsträngige Nukleinsäure-Sequenz erzeugt wird, die über 100.000 Basenpaare lang sein kann.

Die verfahrensgemäße Herstellung der hochkomplexen synthetischen Nukleinsäure, die vorzugsweise aus DNA besteht, erfolgt nach folgendem Prinzip: Zunächst werden relativ kurze DNA-Stränge durch in situ Synthese in einer Vielzahl von Reaktionsbereichen auf einem Reaktionsträger synthetisiert. Dies kann beispielsweise mit den in den Patentanmeldungen WO 0012123, DE 199 40 749, WO0013017 und WO 0013018 beschriebenen Trägern geschehen. Dabei ist jeder Reaktionsbereich für die individuelle und spezifische Synthese einer einzelnen vorgegebenen DNA-Sequenz von c.a. 10 - 100 Nukleotiden Länge geeignet. Diese DNA-Stränge bilden die Bausteine für den gezielten Aufbau sehr langer DNA-Moleküle. Dabei kann der verwendete fluidische Mikroprozessor speziell für die Anwendung gestaltete Reaktionsräume tragen.

Die DNA-Synthese selbst erfolgt somit in Anlehnung an die automatisierte Festphasensynthese, jedoch mit einigen neuheitlichen Aspekten: Die "Festphase" ist in diesem Fall ein individueller Reaktionsbereich auf der Oberfläche des Trägers, z.B. die Wand des Reaktionsraums, also nicht in den Reaktionsraum eingebrachte Partikel, wie dies in einem konventionellen Synthesizer der Fall ist. Durch Integration der Synthese in einem mikrofluidischen Reaktionsträger (z.B. eine Struktur mit gegebenenfalls verzweigten Kanälen und Reaktionsräumen) können die Reagenzien und andere Komponenten, wie Enzyme, zugeführt werden.

Nach der Synthese folgt ein Ablösen der synthetisierten Bausteine von diesen Reaktionsbereichen. Dieser Ablöseprozeß kann orts- oder/und zeitspezifisch für einzelne, mehrere oder alle DNA-Stränge durchgeführt werden.

Für eine bevorzugte Verfahrensvariante ist es vorgesehen, mehrere Reaktionsbereiche innerhalb eines fluidischen Raumes bzw. Kompartements zu realisieren und zu nutzen, so daß die dort synthetisierten DNA-Stränge in einem Arbeitsschritt abgelöst und aus dem Kompartement, das die Reaktionsbereiche fluidisch verbindet, abgeführt werden können.

Danach werden geeignete Kombinationen der abgelösten DNA-Stränge gebildet. Die Zusammenlagerung von einzelsträngigen oder/und doppelsträngigen Bausteinen erfolgt dann beispielsweise innerhalb eines Reaktionsraumes, der einen oder mehrere Reaktionsbereiche für die Synthese beinhalten kann. Die Sequenz der einzelnen Bausteine wird dabei zweckmäßigerweise so gewählt, daß beim Inkontaktbringen der einzelnen Bausteine zueinander komplementäre Bereiche an den beiden zusammengebrachten Enden zur Verfügung stehen, um durch Hybridisierung dieser Bereiche eine spezifische Aneinanderlagerung von weiteren DNA-Strängen zu ermöglichen. Damit entstehen längere DNA-Hybride. Das Phosphordiester-Rückgrat dieser DNA-Hybride kann kovalent, z.B. durch Ligasen, geschlossen und eventuelle Lücken im Doppelstrang in bekannter Vorgehensweise enzymatisch mittels Polymerasen aufgefüllt werden. Eventuell vorhandene einzelsträngige Bereiche können durch Enzyme (z.B. Klenow-Fragment) unter Zugabe von geeigneten Nukleotiden aufgefüllt werden. So entstehen längere DNA-Moleküle. Durch die Zusammenführung von Clustern an derart synthetisierten DNA-Strängen innerhalb von Reaktionsräumen können wiederum längere Teilsequenzen des finalen DNA-Moleküles erzeugt werden. Dies kann stufenweise geschehen, und die Teilsequenzen werden dabei zu immer längeren DNA-Molekülen zusammengesetzt. Auf diese Weise lassen sich sehr lange DNA-Sequenzen als vollsynthetisches Molekül mit einer Länge von über 100.000 Basenpaaren erzeugen.

Die Menge an individuellen Bausteinen, die für ein langes synthetisches DNA-Molekül notwendig ist, wird in dem Reaktionsträger durch parallele Synthese der Bausteine in einem orts- oder/und zeitaufgelösten Synthese-Verfahren bewältigt. Diese parallele Synthese erfolgt in der bevorzugten Ausführungsform durch lichtabhängige orts- oder/und zeitaufgelöste DNA-Synthese in einem fluidischen Mikroprozessor, wie er auch in den Patentanmeldungen WO 0012123, DE 199 40 749, WO 0013017 und WO 0013018 beschrieben ist.

Der miniaturisierte Reaktionsträger bewirkt dabei eine Reduktion der Menge an Einsatzstoffen um mindestens einen Faktor 1.000 im Vergleich zu einem konventionellen DNA-Synthesizer. Gleichzeitig wird eine extreme Vielzahl an Nukleinsäuredoppelsträngen definierter Sequenz hergestellt. Nur so kann die Erzeugung einer sehr großen Vielfalt an individuellen Bausteinen, wie sie für die Synthese langer DNA-Moleküle Voraussetzung ist, unter Einsatz einer ökonomisch sinnvollen Menge an Ressourcen erfolgen. Für den Aufbau einer Sequenz von 100.000 Basenpaaren aus überlappenden Bausteinen von 20 Nukleotiden Länge werden 10.000 individuelle Bausteine benötigt. Dies kann mit entsprechend miniaturisierten Geräten in einem hochparallelen Synthese-Verfahren geleistet werden.

Für die rationelle Bearbeitung genetischer Moleküle und die systematische Erfassung aller möglichen Varianten müssen die Bausteine in ihrer individuellen Sequenz flexibel und ökonomisch hergestellt werden. Dies leistet das Verfahren vorzugsweise durch den Einsatz einer programmierbaren Lichtquellenmatrix für die lichtabhängige orts- oder/und zeitaufgelöste in situ Synthese der DNA-Stränge, die wiederum als Bausteine für den Aufbau längerer DNA-Stränge Verwendung finden können. Diese flexible Synthese erlaubt die freie Programmierung der individuellen Sequenzen der Bausteine und damit auch die Erzeugung von beliebigen Varianten der Teilsequenzen oder der finalen Sequenz, ohne daß damit wesentliche Veränderungen von Komponenten des Systems (Hardware) verbunden wären. Durch diese programmierte Synthese der Bausteine und damit der finalen Syntheseprodukte kann die Vielfalt genetischer Elemente systematisch bearbeitet werden. Gleichzeitig erlaubt die Verwendung der computergesteuert programmierbaren Synthese die Automatisierung des gesamten Prozesses inklusive der Kommunikation mit entsprechenden Datenbanken.

Die Auswahl der Sequenz der einzelnen Bausteine kann bei Vorgabe der Zielsequenz rationell unter Berücksichtigung von biochemischen und funktionellen Parametern erfolgen. Dabei sucht ein Algorithmus nach Eingabe der Zielsequenz (z.B. aus einer Datenbank) die geeigneten Überlappungsbereiche heraus. Je nach der Aufgabenstellung können unterschiedlich viele Teilsequenzen erstellt werden, und zwar innerhalb eines Reaktionsträgers oder auf mehrere Reaktionsträger verteilt. Die Anlagerungsbedingungen für die Bildung der Hybride, wie z.B. Temperatur, Salzkonzentration etc., werden durch einen entsprechenden Algorithmus auf die zur Verfügung stehenden Überlappungsbereiche abgestimmt. So wird eine maximale Spezifität der Aneinanderlagerung gewährleistet. Die Daten für die Zielsequenz können in einer vollautomatischen Version auch direkt aus öffentlichen oder privaten Datenbanken entnommen und in entsprechende Zielsequenzen umgewandelt werden. Die entstehenden Produkte können wiederum optional in entsprechend automatisierte Abläufe eingespeist werden, z.B. in die Klonierung in geeigneten Zielzellen.

Der stufenweise Aufbau durch Synthese der einzelnen DNA-Stränge in Reaktionsbereichen innerhalb umgrenzter Reaktionsräume erlaubt auch den Aufbau schwieriger Sequenzen, z.B. solche mit internen Wiederholungen von Sequenzabschnitten, wie sie z.B. bei Retroviren und entsprechenden retroviralen Vektoren vorkommen. Durch die gesteuerte Ablösung von Bausteinen innerhalb der fluidischen Reaktionsräume kann eine Synthese beliebiger Sequenz erfolgen, ohne daß Probleme durch die Zuordnung der überlappenden Bereiche auf den einzelnen Bausteinen entstehen.

Die hohen Qualitätsanforderungen, die beim Aufbau sehr langer DNA-Moleküle notwendig sind, können u.a. durch die Verwendung einer Echtzeit-Qualitätskontrolle erfüllt werden. Dabei wird die ortsaufgelöste Synthese der Bausteine überwacht, ebenso die Ablösung und die Zusammenlagerung bis hin zur Erstellung der finalen Sequenz. Dann laufen alle Prozesse in einem lichtdurchlässigen Reaktionsträger ab. Desweiteren wird die Möglichkeit geschaffen, im Durchlicht Reaktionen und fluidische Vorgänge durch z.B. eine CCD-Detektion zu verfolgen.

Der miniaturisierte Reaktionsträger wird vorzugsweise so ausgeführt, daß ein Ablösevorgang in den einzelnen Reaktionsräumen möglich ist, und somit die auf den innerhalb dieser Reaktionsräume liegenden Reaktionsbereichen synthetisierten DNA-Stränge einzeln oder in Clustern abgelöst werden. Bei geeigneter Ausführung des Reaktionsträgers ist die Zusammenlagerung der Bausteine in einem stufenweisen Prozeß in Reaktionsräumen möglich, außerdem die Entnahme von Bausteinen, Teilsequenzen oder des finalen Produktes, oder auch die Sortierung bzw. Auftrennung der Moleküle.

Die Zielsequenz kann nach ihrer Fertigstellung als integriertes genetisches Element durch Transfer in Zellen eingebracht und dadurch kloniert und im Zuge funktioneller Studien untersucht werden. Eine weitere Möglichkeit ist es, das Syntheseprodukt zuerst weiter aufzureinigen oder zu analysieren, wobei diese Analyse z.B. eine Sequenzierung sein kann. Der Prozeß der Sequenzierung kann auch durch direkte Kopplung mit einem entsprechenden Gerät beginnen, z.B. mit einer nach dem in den Patentanmeldungen WO 0012123, DE 199 40 749, WO 0013017 und WO 0013018 beschriebenen Vorrichtung zur integrierten Synthese und Analyse von Polymeren. Es ist ebenfalls denkbar, die erzeugten Zielsequenzen nach der Klonierung zu isolieren und zu analysieren.

Das erfindungsgemäße Verfahren stellt über die damit erzeugten integrierten genetischen Elemente ein Werkzeug zur Verfügung, das für die Weiterentwicklung der molekularen Biologie die biologische Vielfalt in einem systematischen Prozeß erfaßt. Die Erzeugung von DNA-Molekülen mit gewünschter genetischer Information ist damit nicht mehr der Engpaß molekularbiologischer Arbeiten, da von kleinen Plasmiden über komplexe Vektoren bis zu Mini-Chromosomen alle Moleküle synthetisch erzeugt werden können und für weitere Arbeiten zur Verfügung stehen.

Das Herstellungsverfahren erlaubt die Erzeugung von zahlreichen verschiedenen Nukleinsäuren und damit einen systematischen Ansatz für Fragestellungen betreffend Regulationselemente, DNA-Bindestellen für Regulatoren, Signalkaskaden, Rezeptoren, Wirkung und Interaktionen von Wachstumsfaktoren etc.

Durch die Integration von genetischen Elementen in eine vollsynthetische Gesamt-Nukleinsäure können die bekannten genetischen Werkzeuge wie Plasmide und Vektoren weiter genutzt und es kann so auf die entsprechenden Erfahrungen aufgebaut werden. Andererseits werden sich diese Erfahrungen durch die angestrebte Optimierung der vorhandenen Vektoren etc. rasch verändern. Die Mechanismen, die z.B. ein Plasmid für die Propagation in einem bestimmten Zelltyp geeignet machen, lassen sich auf der Grundlage des erfindungsgemäßen Verfahrens erstmals rationell untersuchen.

Durch diese rationelle Untersuchung großer Varianten-Zahlen läßt sich der gesamte Kombinationsraum genetischer Elemente erschließen. Damit wird neben die zur Zeit in rascher Entwicklung befindliche hochparallele Analytik (u.a. auf DNA-Arrays oder DNA-Chips) als zweites wichtiges Element die programmierte Synthese integrierter genetischer Elemente geschafffen. Nur beide Elemente zusammen können das Fundament einer rationellen molekularen Biologie bilden.

Bei der programmierten Synthese von entsprechenden DNA-Molekülen ist nicht nur die beliebige Zusammensetzung der codierenden Sequenzen und funktionellen Elemente möglich, sondern auch die Anpassung der Zwischenbereiche. Dies kann rasch zu Minimal-Vektoren und Minimal-Genomen führen, womit wiederum Vorteile durch deren geringere Größe entstehen. Übertragungsvehikel wie z.B. virale Vektoren können dadurch effizienter gemacht werden, z.B. bei Verwendung von retroviralen oder adenoviralen Vektoren.

Über die Kombination bekannter genetischer Sequenzen hinaus ist die Entwicklung neuer genetischer Elemente möglich, die auf der Funktion vorhandener Elemente aufbauen können. Gerade für solche Entwicklungsarbeiten ist die Flexibilität des Systems von enormem Wert.

Die synthetischen DNA-Moleküle sind dabei auf jeder Stufe der Entwicklung des hier beschriebenen Verfahrens voll kompatibel mit der vorhandenen Rekombinationstechnologie. Auch für "traditionelle" molekularbiologische Anwendungen können integrierte genetische Elemente bereitgestellt werden, z.B. durch entsprechende Vektoren. Der Einbau entsprechender Schnittstellen auch für bisher wenig verwendete Enzyme ist bei integrierten genetischen Elementen kein limitierender Faktor.

### Verbesserungen gegenüber dem Stand der Technik

Ermöglicht wird mit diesem Verfahren die Integration aller gewünschten funktionellen Elemente als "genetische Module", wie z.B. Gene, Teile von Genen, Regulationselemente, virale Verpackungssignale etc., in das synthetisierte Nukleinsäuremolekül als Träger genetischer Information. Durch diese Integration ergeben sich u.a. folgende Vorteile:

Es können damit hochgradig funktionsintegrierte DNA-Moleküle entwickelt werden, wobei unnötige DNA-Bereiche wegfallen (Minimal-Gene, Minimal-Genome).

Die freie Kombination der genetischen Elemente sowie die Veränderungen der Sequenz wie z.B. zur Anpassung an den exprimierenden Organismus bzw. Zelltyp (Codon-Nutzung) werden ebenso ermöglicht wie Veränderungen der Sequenz zur Optimierung funktioneller genetischer Parameter, wie beispielsweise Genregulation.

Ebenfalls ermöglicht werden Veränderungen der Sequenz zur Optimierung funktioneller Parameter des Transkriptes, z.B. Spleissen, Regulation auf mRNA-Ebene, Regulation auf Translationsebene, und darüber hinaus die Optimierung funktioneller Parameter des Genprodukts, wie z.B. die Aminosäuresequenz (z.B. Antikörper, Wachstumsfaktoren, Rezeptoren, Kanäle, Poren, Transporter etc.).

Insgesamt ist das mit dem Verfahren realisierte System extrem flexibel und erlaubt in bisher nicht vorhandener Weise die programmierte Erstellung von genetischem Material mit stark verringertem Aufwand an Zeit, Materialien und Arbeit.

Größere DNA-Moleküle, wie z.B. Chromosomen, von mehreren Hundert kbp waren mit den vorhandenen Methoden nahezu nicht gezielt manipulierbar. Bereits komplexere (d.h. größere) virale Genome von mehr als 30 kbp (z.B. Adenoviren) sind mit den klassischen Methoden der Gentechnik schwierig zu handhaben und zu manipulieren.

Durch das erfindungsgemäße Verfahren kommt es zu einer erheblichen Verkürzung bis zur letzten Stufe der Klonierung eines Gens: Das Gen oder die Gene werden als DNA-Molekül synthetisiert und dann (nach geeigneter Vorbereitung wie Reinigung etc.) direkt in Ziel-Zellen eingebracht und das Ergebnis studiert. Der mehrstufige, meist über Mikroorganismen wie E.coli laufende Klonierungsprozeß (z.B. DNA-Isolation, Reinigung, Analyse, Rekombination, Klonierung in Bakterien, Isolation, Analyse etc.) wird damit auf die letzte Übertragung des DNA-Moleküls in die finalen Effektorzellen reduziert. Bei synthetisch hergestellten Genen oder Genfragmenten ist eine klonale Vermehrung in einem Zwischenwirt (zumeist E. coli) nicht mehr notwendig. Damit umgeht man die Gefahr, daß das für die Zielzelle bestimmte Genprodukt eine toxische Wirkung auf den Zwischenwirt ausübt. Damit hebt man sich deutlich ab von der Toxizität mancher Genprodukte, die bei der Verwendung klassischer Plasmid-Vektoren häufig zu erheblichen Problemen bei der Klonierung der entsprechenden Nukleinsäure-Fragmente führt.

Eine weitere erhebliche Verbesserung ist die Zeitverkürzung und die Reduktion der Arbeitsschritte bis nach dem Sequenzieren von genetischem Material, wobei vorgefundene potentielle Gene als solche verifiziert und kloniert werden. Normalerweise werden nach Auffinden interessierender Muster, die als offener Leserahmen (ORF) in Frage kommen, mit Sonden (z.B. mittels PCR) in cDNA-Bibliotheken entsprechende Klone gesucht, die allerdings nicht die ganze Sequenz der ursprünglich bei ihrer Herstellung verwendeten mRNA enthalten müssen. Bei anderen Verfahren wird mittels eines Antikörpers in einer Expressions-Genbibliothek gesucht (Screening).

Beide Verfahren lassen sich mit dem erfindungsgemäßen Verfahren extrem abkürzen: Bei Vorliegen einer "in silico" bestimmten Gen-Sequenz (d.h. nach der Erkennung eines entsprechenden Musters in einer DNA-Sequenz durch den Computer) oder nach Entschlüsselung einer Proteinsequenz kann ein entsprechender Vektor mit der Sequenz oder Varianten davon direkt über programmierte Synthese eines integrierten genetischen Elementes erzeugt und in geeignete Zielzellen eingebracht werden.

Die so erfolgende Synthese von DNA-Molekülen bis zu mehreren 100 kbp erlaubt die direkte Komplett-Synthese von viralen Genomen, z.B. Adenoviren. Diese sind ein wichtiges Werkzeug in Grundlagenforschung (u.a. Gentherapie), aber wegen der Größe ihres Genoms (ca. 40 kbp) schwer mit klassischen Methoden der Gentechnik zu handhaben. Besonders die rasche und ökonomische Erzeugung von Varianten zur Optimierung ist dadurch stark limitiert. Diese Limitierung wird durch das erfindungsgemäße Verfahren aufgehoben.

Durch das Verfahren erfolgen die Integration der Synthese, die Ablösung der Syntheseprodukte und die Zusammenlagerung zu einem DNA-Molekül in einem System. Mit Herstellungsverfahren der Mikrosystemtechnik können alle notwendigen Funktionen und Verfahrensschritte bis zur Aufreinigung des finalen Produktes in einem miniaturisierten Reaktionsträger integriert werden. Dies können Synthesebereiche, Ablösungsbereiche (Cluster), Reaktionsräume, Zuführungskanäle, Ventile, Pumpen, Konzentratoren, Auftrennungsbereiche etc. sein.

Plasmide und Expressionsvektoren können für sequenzierte Proteine oder entsprechende Teilsequenzen direkt hergestellt und die Produkte biochemisch und funktionell analysiert werden, z.B. unter Verwendung geeigneter Regulationselemente. Damit fällt die Suche nach Klonen in einer Gen-Bibliothek weg. Entsprechend können ORFs aus Sequenzierarbeiten (z.B. Humangenomprojekt) direkt in entsprechende Vektoren programmiert und mit gewünschten genetischen Elementen kombiniert werden. Eine Identifikation von Klonen z.B. in durch aufwendiges Screening von cDNA-Bibliotheken entfällt. Damit ist der Informationsfluß von der Sequenz-Analyse zur Funktions-Analyse stark verkürzt worden, denn am selben Tag, an dem ein ORF durch Analyse von Primärdaten im Computer vorliegt, kann ein entsprechender Vektor inklusive des vermuteten Gens synthetisiert und zur Verfügung gestellt werden.

Gegenüber konventioneller Festphasen-Synthese zur Gewinnung synthetischer DNA zeichnet sich das Verfahren gemäß Erfindung durch geringeren Materialaufwand aus. Zur Herstellung tausender von unterschiedlichen Bausteinen für die Erzeugung von einem komplexen integrierten genetischen Element mit mehreren 100.000 kbp Länge, in entsprechend parallelisiertem Format und bei entsprechender Miniaturisierung (siehe Ausführungsbeispiele), braucht ein mikrofluidisches System deutlich weniger Einsatzstoffe als ein konventioneller Festphasensynthese-Automat für ein einzelnes DNA-Oligomer (bei Verwendung einer einzigen Säule). Hier stehen Mikroliter dem Verbrauch von Millilitern gegenüber, d.h. ein Faktor von 1.000.

Unter Berücksichtigung neuester Erkenntnisse der Immunologie erlaubt das vorgestellte Verfahren ein extrem rationelles und schnelles Impfstoff-Design (DNA-Vakzine).

### Ausführungsbeispiele

Die vorliegende Erfindung setzt zur Umsetzung des Verfahrens die Bereitstellung einer großen Anzahl von Nukleinsäure-Molekülen, meist DNA, voraus, die in ihrer Sequenz frei bestimmbar sind. Diese Bausteine müssen innerhalb einer Baustein-Spezies nahezu 100 % identische Sequenz haben (analog der Syntheseleistung konventioneller Syntheseautomaten). Zur Erzeugung der notwendigen Varianz kommen nur hochparallele Syntheseverfahren in Frage. Damit das System flexibel arbeiten kann und dabei trotz der notwendigen Vielzahl von unterschiedlichen zu synthetisierenden Bausteinen möglichst wenig Raum und Reagenzien benötigt, erfolgt die Realisierung vorzugsweise in einem mikrofluidischen System, innerhalb dessen die einzelnen Sequenzen bestimmbar entstehen. Für solche Systeme bieten sich zwei Arten der programmierten Synthese an, wie sie auch in den Patentanmeldungen WO 012123, DE 199 40 749, WO 0013017 und WO 0013018 beschrieben sind: Dies ist einmal die Synthese durch programmierbare fluidische Individualisierung der Reaktionsbereiche und zum anderen die Synthese durch programmierbare lichtabhängige Individualisierung der Reaktionsbereiche.

Bei beiden Varianten wird die Synthese in einem mikrofluidischen Reaktionsträger durchgeführt. Das Design dieses Reaktionsträgers kann dabei im System die stufenweise Zusammenführung der abgelösten Syntheseprodukte, d.h. Bausteine, vorsehen, indem nach dem Ablösen der Nukleinsäurestränge diese in entsprechenden Reaktionsbereichen gesammelt werden und dort die Zusammenlagerung stattfindet. Gruppen solcher Zusammenlagerungs-Bereiche können dann ihrerseits wieder miteinander in Kontakt gebracht werden, so daß im Laufe einer mehr oder weniger langen Kaskade die finalen Produkte der Synthese entstehen: Gentechnische Informationsträger in Form von DNA-Molekülen. Die folgenden Varianten kommen dabei in Frage:
Entweder erfolgen Synthese, Ablösung und Zusammenlagerung zeitlich nacheinander, aber räumlich integriert in einem mikrofluidischen Reaktionsträger, oder aber Synthese, Ablösung und Zusammenlagerung erfolgen teilweise parallel in einem oder mehreren mikrofluidischen Reaktionsträgern. Weiterhin ist es möglich, daß der mikrofluidische Reaktionsträger nur Reaktionsbereiche für die programmierte Synthese enthält und anschließend die Ablösung und Eluation in ein Reaktionsgefäß für die Zusammenlagerung erfolgt.

Synthese, Ablösung und Zusammenlagerung können bei sehr großen DNA-Molekülen durch Kondensations-Strategien ergänzt werden, die ein Zerbrechen der Moleküle verhindern. Dazu zählt z.B. die Verwendung von Histonen (Kernproteine, die bei Eukaryoten die Kondensation der Chromosomen im Zellkem ermöglichen), der Einsatz von Topoisomerasen (Enzyme für die Verdrillung von DNA in Eukaryoten und Prokaryoten) oder die Zugabe von anderen DNA-bindenden, stabilisierenden und kondensierenden Agenzien bzw. Proteinen. Dies kann je nach Design des Reaktionsträgers durch Integration der Kondensationsreaktion in einer weiteren dafür vorgesehenen Reaktionskammer geschehen oder durch Zugabe während der stufenweisen Kombination und Zusammenlagerung der Bausteine.

Die freie Wahl der Sequenz ist von wesentlicher Bedeutung für die kontrollierte und effiziente stufenweise Zusammenlagerung der Bausteine bis zum Endprodukt. Denn durch die Wahl der überlappenden komplementären Enden werden die Spezifität der Zusammenlagerung und die biochemischen Rahmenbedingungen (Salzkonzentration, Temperatur etc.) beeinflußt. Bei Vorgabe einer Sequenz für das interessierende Gen und nach automatischer oder manueller Auswahl der anderen genetischen Elemente (Regulationsbereiche, Resistenzgene zum Klonieren, Propagationssignale etc.) zur Bestimmung des finalen Produktes (z.B. ein Plasmid-Vektor) zerlegt man die vorgegebene Sequenz in geeignete Bausteine und synthetisiert diese in der notwendigen Zahl von Reaktionsträgern. Die Fragmente bzw. deren zu hybridisierende Überlappungsbereiche werden so gewählt, daß die Bedingungen für das Hybridisieren möglichst ähnlich sind (u.a. GC : AT Verhältnis, Schmelzpunkte, etc.).

Bei weiterem Ausbau des Systems sind ebenfalls in Mikrofluidik bzw. Mikrosystemtechnik ausgeführte Elemente für die Reinigung und Isolation des entstehenden Produktes vorgesehen. Dies können z.B. Verfahren sein, bei denen die finale doppelsträngige DNA nach ihrem Aufbau unter Verwendung von fluoreszierenden Synthonen eine bestimmte Gesamtfluoreszenz aufweisen muß. Gegebenenfalls kann bei Einsatz von kondensierend wirkenden Proteinen auch an diesen eine Fluoreszenzmarkierung hängen, die vorzugsweise getrennt detektierbar ist (Referenzsignal), In den Reaktionsträger-Strukturen kann dann das Gemisch an finalem Reaktionsprodukt nach Fluoreszenz sortiert werden (siehe Chou et al., Proceedings of the National Academy of Science PNAS 96:11-13, 1999). Somit wird eine ausreichende Qualität erreicht, um für die weiteren Arbeiten direkt ein Produkt zur Verfügung zu stellen.

Informationen aus Sequenzierprojekten, die in Datenbanken vorliegen, können (computergestützt) vollautomatisch auf Gene hin untersucht werden. Identifizierte oder vermutete Gene (ORFs) werden in vollsynthetische DNA umgewandelt, die gegebenenfalls geeignet erscheinende regulatorische und sonstige genetische Elemente enthalten kann, so daß z.B. ein oder mehrere Vektoren erzeugt werden. Das Produkt wird entweder zur Verfügung gestellt (z.B. als reine DNA) oder direkt in funktionelle Untersuchungen eingespeist, u.a. durch Transfer in geeignete Zielzellen. Die Information kann aus öffentlichen Datenbanken, aus Arbeiten dezentraler Anwender oder aus sonstigen Quellen, z.B. dem in den Patentanmeldungen WO 0012123 und DE 199 40 749 beschriebenen Verfahren stammen.

Es kann von Interesse sein, daß an bestimmter Stelle oder Stellen der Zielsequenz eine Varianz an randomisierter Sequenz vorkommt. Ein Beispiel ist das Austesten von Varianten einer Bindestelle, bei der z.B. über einen Bereich von 20 Aminosäuren, d.h. 60 Nukleotiden, zufällige Variationen an Nuldeotiden eingebaut wurden. Dies kann in einer Ausführungsform geschehen, indem während des Syntheseprozesses nach Aktivierung eines Reaktionsbereiches ein Gemisch an Synthonen zugeführt wird, so daß sich alle zugegebenen Synthone statistisch verteilt anlagern können. Eine Abwandlung dieses Prozesses kann vorsehen, daß an einer bestimmten Position der Zielsequenz unterschiedlich lange DNA-Bausteine eingesetzt werden, z.B. indem unterschiedliche Bausteine auf unterschiedlichen Reaktionsbereichen hergestellt werden, die die gleiche Sequenz zur Überlappung und Hybridisierung zeigen.

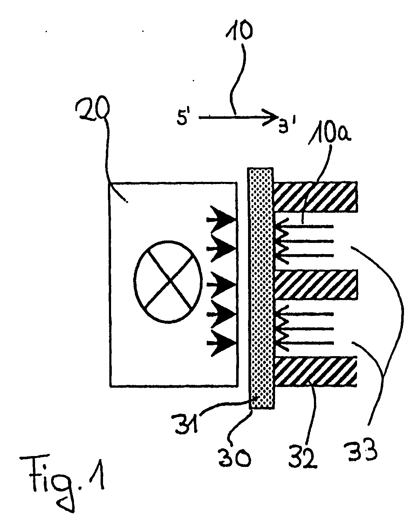

Fig. 1 zeigt einen Reaktionsträger 30 im senkrechten Schnitt orthogonal zu den darauf befindlichen Mikrokanälen 33, die durch Wände 32 voneinander getrennt sind. Die Unterseite 31 des Reaktionsträgers ist dabei lichtdurchlässig. Weiterhin schematisch dargestellt ist eine einzelsträngige Nukleinsäure 10 mit der konventionsgerechten Bezeichnung des 5'- und des 3'-Endes. Diese sind dargestellt als 10a mit dem 3'-Ende am Reaktionsträger 30 kovalent angebunden durch Festphasensynthese. Ebenfalls dargestellt ist eine Lichtquellenmatrix 20 mit einer Lichtquelle und dem Reaktionsträger 30 zugewandten steuerbaren Belichtungsaustritt.

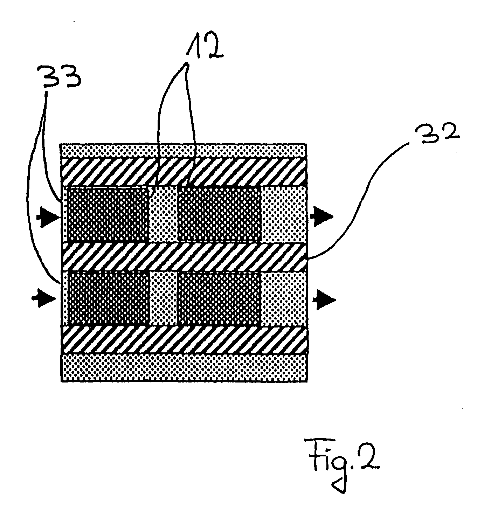

Fig. 2 zeigt einen Reaktionsträger 30 in Draufsicht mit Reaktionsbereichen 12 und den Wänden 32 zwischen den Mikrokanälen 33. Die Pfeile veranschaulichen die Durchflußrichtung.

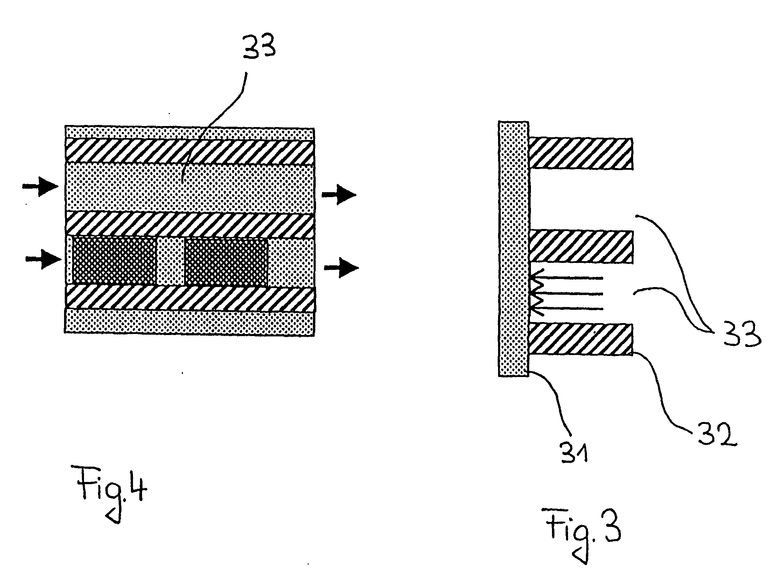

Fig.3 zeigt ähnlich wie Fig.1 einen senkrechten Schnitt durch den Reaktionsträger 30, wobei im Mikrokanal 33 die einzelsträngigen Nukleinsäuren abgelöst sind.

In Fig. 4 ist wiederum der Reaktionsträger 30 in Draufsicht dargestellt, wobei im Mikrokanal 33 die einzelsträngigen Nukleinsäuren abgelöst sind.

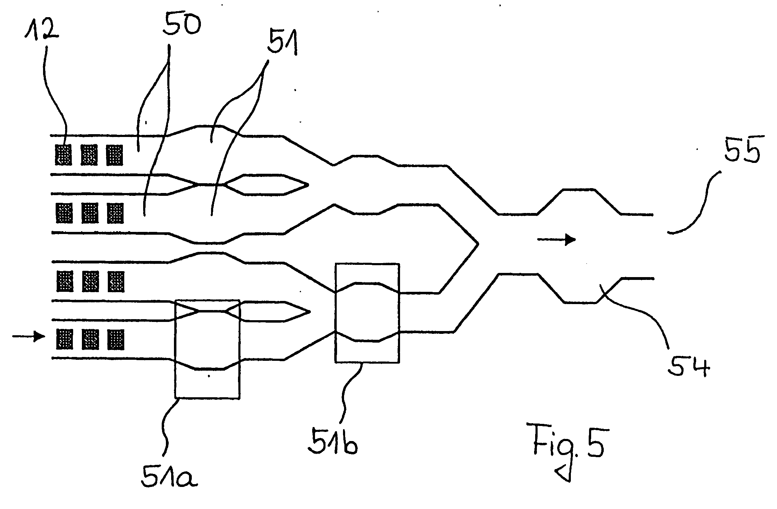

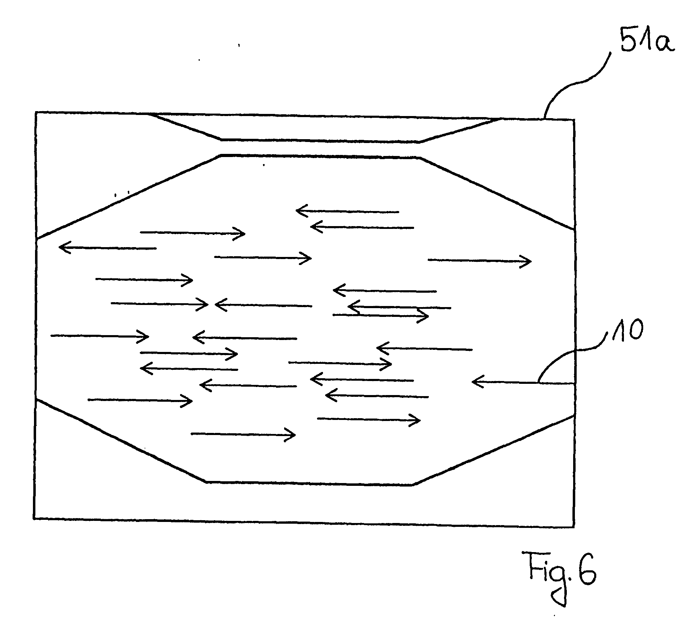

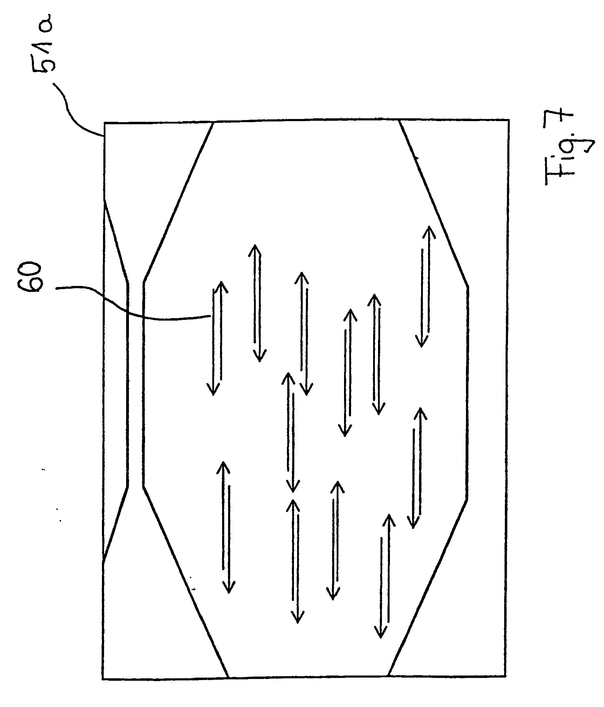

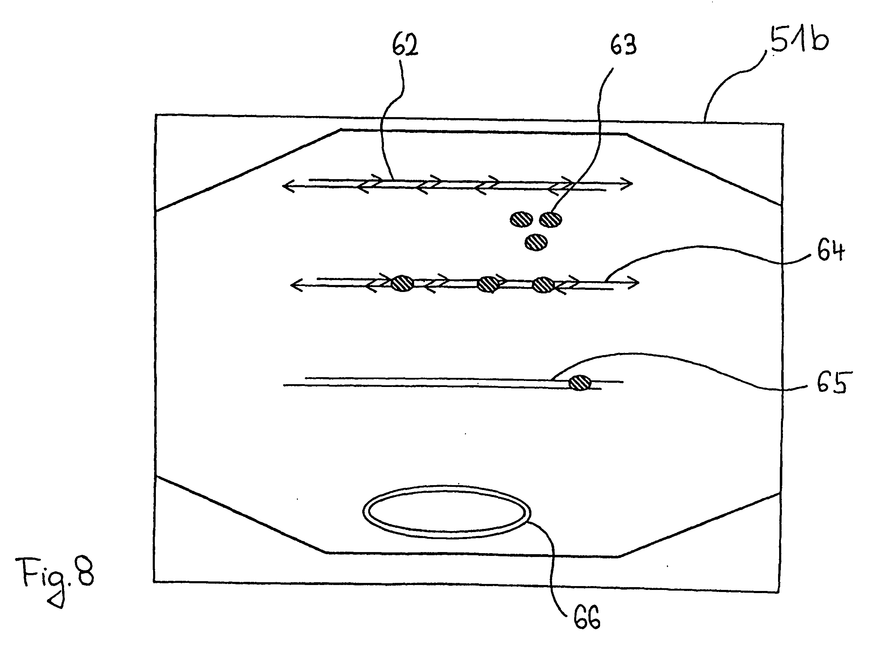

Fig. 5 zeigt die Anordnung der Mikrokanäle in Aufsicht mit fluidischen Reaktionsräumen 50, die die einzelnen Reaktionsbereiche enthalten, und Reaktionskammern, wo die Zusammenlegung einer Teilsequenz erfolgt. Im Reaktionsraum 54 werden alle Mikrokanäle innerhalb eines Reaktionsträgers zusammengeführt. Dort findet auch die Zusammenlagerung des finalen Syntheseprodukts statt, welches durch den Ausgang 55 entnommen wird. Die Bezugszeichen 51 a und 51 b kennzeichnen die in Fig.6 und Fig.7 bzw. Fig.8 dargestellten vergrößerten Darstellungen einer Reaktionskammer. Die Pfeile signalisieren wiederum die Flußrichtung.

Fig. 6 zeigt eine vergrößerte Darstellung einer Reaktionskammer 51 a nach einem Mikrokanal mit abgelösten einzelsträngigen Nukleinsäuren.

Fig. 7 zeigt eine vergrößerte Darstellung einer Reaktionskammer 51 a nach einem Mikrokanal mit einem doppelsträngigen Hybrid 60 aus zwei aneinandergelagerten komplementären Nukleinsäureeinzelsträngen.

Fig. 8 zeigt eine vergrößerte Darstellung einer Reaktionskammer 51 b nach Zusammenführung von zwei Mikrokanälen mit einem zusammengelagerten doppelsträngigen Nukleinsäure-Hybrid 62, Enzyme 63 (z.B. Ligasen) für die kovalente Verknüpfung der Bausteine des Nukleinsäure-Hybrids 85, einem linearen kovalent verknüpften Nukleinsäuredoppelstrang 65 und einem ringförmig geschlossenen Nukleinsäuredoppelstrang 66 (z.B. Vektor).

Als Bezugszeichen 64 ist eine Reaktion der Enzyme mit dem Nukleinsäure-Hybrid dargestellt.

## Patentansprüche

1. Verfahren zur Synthese von Polymeren,
**dadurch gekennzeichnet,**
**daß** eine Vielzahl von Oligomerbaublöcken auf jeweils unterschiedlichen Bereichen eines gemeinsamen Trägers durch parallele Syntheseschritte aufgebaut, vom Träger abgelöst und untereinander zum Aufbau des Polymeren in Kontakt gebracht werden.

2. Verfahren nach Anspruch 1,
**dadurch gekennzeichnet,**
**daß** doppelsträngige Nukleinsäure-Polymere mit einer Länge von mindestens 300 bp, insbesondere mindestens 1000 bp synthetisiert werden.

3. Verfahren nach Anspruch 1 oder 2,
**dadurch gekennzeichnet,**
**daß** Nukleinsäure-Polymere ausgewählt aus Genen, Genclustern, Chromosomen, viralen und bakteriellen Genomen oder Abschnitten davon synthetisiert werden.

4. Verfahren nach einem der Ansprüche 1 bis 3,
**dadurch gekennzeichnet,**
**daß** die Oligomerbaublöcke eine Länge von 5 bis 150, vorzugsweise 5 bis 30 Monomereinheiten aufweisen.

5. Verfahren nach einem der Ansprüche 1 bis 4,
**dadurch gekennzeichnet,**
**daß** in aufeinanderfolgenden Schritten jeweils partiell komplementäre Oligonukleotidbaublöcke vom Träger abgelöst und unter Hybridisierungsbedingungen miteinander bzw. mit dem Polymer-Zwischenprodukt in Kontakt gebracht werden.

6. Verfahren nach einem der Ansprüche 1 bis 5 zur Herstellung synthetischer Nukleinsäuredoppelstränge wahlfreier Sequenz, umfassend die Schritte:
(a) Bereitstellen eines Trägers mit einer Oberfläche, die eine Vielzahl von individuellen Reaktionsbereichen enthält,
(b) ortsaufgelöstes Synthetisieren von Nukleinsäurefragmenten mit jeweils unterschiedlicher Basensequenz an mehreren der individuellen Reaktionsbereiche, und
(c) Ablösen der Nukleinsäurefragmente von individuellen Reaktionsbereichen.

7. Verfahren nach Anspruch 6,
**dadurch gekennzeichnet,**
**daß** die Basensequenzen der an individuellen Reaktionsbereichen synthetisierten Nukleinsäurefragmente so gewählt werden, daß sie sich zu einem Nukleinsäuredoppelstrang-Hybrid zusammenlagern können.

8. Verfahren nach Anspruch 6 oder 7,
**dadurch gekennzeichnet,**
**daß** das Ablösen der Nukleinsäurefragmente gemäß Schritt (c) in einem oder mehreren Schritten unter solchen Bedingungen erfolgt, daß sich mehrere der abgelösten Nukleinsäurefragmente zu einem Nukleinsäuredoppelstrang-Hybrid zusammenlagern.

9. Verfahren nach Anspruch 8,
**dadurch gekennzeichnet,**
**daß** mehrere Nukleinsäurefragmente, die einen Strang des Nukleinsäuredoppelstrang-Hybrids bilden, kovalent miteinander verbunden werden.

10. Verfahren nach Anspruch 9,
**dadurch gekennzeichnet,**
**daß** das kovalente Verbinden eine Behandlung mit Ligase oder/und ein Auffüllen von Lücken in den Strängen mit DNA-Polymerase umfaßt.

11. Verfahren nach einem der Ansprüche 6 bis 10,
**dadurch gekennzeichnet,**
**daß** in der Sequenz an einer oder mehreren Positionen Erkennungssequenzen für die spezifische Interaktion mit Molekülen wie Proteinen, Nukleinsäuren, Peptiden, Pharmaka, Sacchariden, Lipiden, Hormonen oder/und organischen Verbindungen enthalten sind.

12. Verfahren nach einem der Ansprüche 6 bis 11,
**dadurch gekennzeichnet,**
**daß** die Sequenz der Nukleinsäuredoppelstränge eine natürlich vorkommende Sequenz, eine nicht natürlich vorkommende Sequenz oder eine Kombination aus diesen beiden ist.

13. Verfahren nach einem der Ansprüche 6 bis 12,
**dadurch gekennzeichnet,**
**daß** die Sequenz aus einer Datenbank, einem Sequenzierexperiment oder einer Vorrichtung zur integrierten Synthese und Analyse von Polymeren entnommen ist.

14. Verfahren nach einem der Ansprüche 1 bis 13,
**dadurch gekennzeichnet,**
**daß** die Oligomerbaublöcke durch orts- oder/und zeitaufgelöste Belichtung mittels einer programmierbaren Lichtquellenmatrix synthetisiert werden.

15. Verfahren nach einem der Ansprüche 1 bis 14,
**dadurch gekennzeichnet,**
**daß** eine orts- oder/und zeitaufgelöste Synthese der Oligomerbaublöcke in einem mikrofluidischen Reaktionsträger mit einem oder mehreren fluidischen Reaktionsräumen und einem oder mehreren Reaktionsbereichen innerhalb eines fluidischen Reaktionsraumes erfolgt.

16. Verfahren nach einem der Ansprüche 1 bis 15,
**dadurch gekennzeichnet,**
**daß** die Synthesebausteine in der Natur vorkommende Nukleotide, modifizierte Nukleotide oder Mischungen davon enthalten.

17. Verfahren nach einem der Ansprüche 1 bis 16,
**dadurch gekennzeichnet,**
**daß** modifizierte Synthesebausteine zur Markierung und späteren Detektion der zusammengelagerten Nukleinsäuredoppelstränge verwendet werden.

18. Verfahren nach Anspruch 17,
**dadurch gekennzeichnet,**
**daß** als Markierungsgruppen lichtabhängig zu detektierende Moleküle verwendet werden.

19. Verfahren nach einem der Ansprüche 1 bis 18 zur Herstellung eines Nukleinsäuredoppelstranges wahlfreier Sequenz als therapeutisches oder pharmakologisches Mittel.

20. Verfahren nach einem der Ansprüche 1 bis 18 zur Herstellung eines Nukleinsäuredoppelstranges wahlfreier Sequenz als diagnostisches Mittel.

21. IN VITRO Verfahren nach Anspruch 19 oder 20, umfassend die direkte Zuführung des hergestellten Nukleinsäuredoppelstranges zum beabsichtigten Zweck.

22. IN VITRO Verfahren nach Anspruch 19 oder 20, umfassend eine Umsetzung in Effektorzellen.

23. Verfahren nach einem der Ansprüche 1 bis 18 zur Herstellung eines Nukleinsäuredoppelstranges wahlfreier Sequenz, wobei dieser während der stufenweisen Kombination und Zusammenlagerung oder im Anschluß daran stabilisiert, kondensiert oder/und topologisch manipuliert wird.

24. Verfahren nach Anspruch 23, wobei die Stabilisierung, Kondensation oder/und topologische Manipulierung durch funktionelle Moleküle wie Histone oder Topoisomerasen erfolgt.

25. Verfahren nach einem der Ansprüche 1 bis 18 zur Herstellung eines Nukleinsäuredoppelstranges als propagierbarer Klonierungs-Vektor, wobei der propagierbare Klonierungs-Vektor in geeigneten Zielzellen der Transkription, der Expression der transkribierten Sequenz, und gegebenenfalls der Gewinnung von exprimierten Genprodukten dienen kann.

## Claims

1. Process for synthesizing polymers,
**characterized in that**
a plurality of oligomer building blocks are synthesized on different areas of a common support by parallel synthesis steps, are detached from the support and brought into contact with one another to synthesize the polymer.

2. Process according to claim 1,
**characterized in that**
double-stranded nucleic acid polymers having a length of at least 300 bp and in particular of at least 1000 bp are synthesized.

3. Process according to claim 1 or 2,
**characterized in that**
nucleic acid polymers selected from genes, gene clusters, chromosomes, viral and bacterial genomes or sections thereof are synthesized.

4. Process according to one of the claims 1 to 3,
**characterized in that**
the oligomer building blocks are from 5 to 150, preferably 5 to 30 monomer units in length.

5. Process according to one of the claims 1 to 4,
**characterized in that**
in each case partially complementary oligonucleotide building blocks are detached from the support in successive steps and are brought into contact with one another or with the polymer intermediate under hybridization conditions.

6. Process according to one of the claims 1 to 5 for producing synthetic nucleic acid double strands of optional sequence, comprising the steps:
(a) provision of a support with a surface which contains a plurality of individual reaction areas,
(b) spatially-resolved synthesis of nucleic acid fragments each having a different base sequence in several of the individual reaction areas, and
(c) detaching the nucleic acid fragments from individual reaction areas.

7. Process according to claim 6,
**characterized in that**
the base sequences of the nucleic acid fragments synthesized in individual reaction areas are selected such that they can associate to form a nucleic acid double strand hybrid.

8. Process according to claim 6 or 7,
**characterized in that**
the nucleic acid fragments according to step (c) are detached in one or more steps under conditions such that several of the detached nucleic acid fragments associate to form a nucleic acid double strand hybrid.

9. Process according to claim 8,
**characterized in that**
several nucleic acid fragments which form one strand of the nucleic acid double strand hybrid are linked covalently to one another.

10. Process according to claim 9,
**characterized in that**
the covalent linking includes treatment with ligase or/and filling in gaps in the strands using DNA polymerase.

11. Process according to one of the claims 6 to 10,
**characterized in that**
the sequence contains recognition sequences at one or more positions for specific interaction with molecules such as proteins, nucleic acids, peptides, pharmaceuticals, saccharides, lipids, hormones or/and organic compounds.

12. Process according to one of the claims 6 to 11,
**characterized in that**
the sequence of the nucleic acid double strands is a naturally occurring sequence, a non-naturally occurring sequence or a combination of these two.

13. Process according to one of the claims 6 to 12,
**characterized in that**
the sequence is taken from a database, a sequencing experiment or a device for the integrated synthesis and analysis of polymers.

14. Process according to one of the claims 1 to 13,
**characterized in that**
the oligomer building blocks are synthesized by spatially-resolved or/and time-resolved exposure using a programmable light source matrix.

15. Process according to one of the claims 1 to 14,
**characterized in that**
a spatially-resolved or/and time-resolved synthesis of the oligomer building blocks takes place in a microfluidic reaction support having one or more fluidic reaction spaces and one or more reaction areas within a fluidic reaction space.

16. Process according to one of the claims 1 to 15,
**characterized in that**
the synthesis building blocks contain naturally occurring nucleotides, modified nucleotides or mixtures thereof.

17. Process according to one of the claims 1 to 16,
**characterized in that**
the modified synthesis building blocks are used to label and subsequently detect the associated nucleic acid double strands.

18. Process according to claim 17,
**characterized in that**
molecules which are to be detected in a light-dependent manner are used as labelling groups.

19. Process according to one of the claims 1 to 18 to produce a nucleic acid double strand of optional sequence as a therapeutic or pharmacological agent.

20. Process according to one of the claims 1 to 18 to produce a nucleic acid double strand of optional sequence as a diagnostic agent.

21. In vitro process according to claim 19 or 20 comprising the direct supply of the synthesized nucleic acid double strand for the intended purpose.

22. In vitro process according to claim 19 or 20 comprising a reaction in effector cells.

23. Process according to one of the claims 1 to 18 for producing a nucleic acid double strand of optional sequence, wherein the said nucleic acid double strand is stabilized, condensed or/and topologically manipulated during or following the stepwise combination and association.

24. Process according to claim 23, wherein the stabilization, condensation or/and topological manipulation is carried out by functional molecules such as histones or topoisomerases.

25. Process according to one of the claims 1 to 18 for producing a nucleic acid double strand as a propagatable cloning vector, wherein the propagatable cloning vector can be used for transcription, expression of the transcribed sequence and optionally for obtaining expressed gene products in suitable target cells.

## Revendications

1. Procédé de synthèse de polymères, **caractérisé en ce que** l'on synthétise une pluralité de blocs structuraux oligomères sur des zones à chaque fois différentes d'un support commun par des étapes de synthèse parallèles, on les sépare du support et on les met en contact entre eux pour synthétiser le polymère.

2. Procédé selon la revendication 1, **caractérisé en ce que** l'on synthétise des polymères d'acides nucléiques double brin ayant une longueur d'au moins 300 pb, en particulier d'au moins 1 000 pb.

3. Procédé selon la revendication 1 ou 2, **caractérisé en ce que** l'on synthétise des polymères d'acides nucléiques choisis parmi des gènes, des groupes de gènes, des chromosomes, des génomes viraux ou bactériens ou leurs fragments.

4. Procédé selon l'une des revendications 1 à 3, **caractérisé en ce que** les blocs structuraux oligomères ont une longueur de 5 à 150, de préférence de 5 à 30 unités monomères.

5. Procédé selon l'une des revendications 1 à 4, **caractérisé en ce que**, dans des étapes successives, on sépare du support des blocs structuraux oligonucléotidiques à chaque fois partiellement complémentaires et on les met en contact entre eux ou avec le produit intermédiaire du polymère dans des conditions d'hybridation.

6. Procédé selon l'une des revendications 1 à 5 pour la préparation d'acides nucléiques double brin synthétiques d'une séquence au choix, comprenant les étapes:
(a) préparation d'un support avec une surface qui contient une pluralité de zones de réactions individuelles,
(b) synthèse séparée dans l'espace de fragments d'acides nucléiques ayant à chaque fois une séquence de bases différente sur plusieurs des zones de réactions individuelles, et
(c) séparation des fragments d'acides nucléiques des zones de réaction individuelles.

7. Procédé selon la revendication 6, **caractérisé en ce que** les séquences de bases des fragments d'acides nucléiques synthétisés sur les zones de réaction individuelles sont choisies de manière qu'elles puissent s'associer en un acide nucléique double brin hybride.

8. Procédé selon la revendication 6 ou 7, **caractérisé en ce que** la séparation des fragments d'acides nucléiques selon l'étape (c) s'effectue en une ou plusieurs étapes dans des conditions dans lesquelles plusieurs des fragments d'acides nucléiques séparés s'associent en un acide nucléique double brin hybride.

9. Procédé selon la revendication 8, **caractérisé en ce que** plusieurs fragments d'acides nucléiques, qui forment un brin de l'acide nucléique double brin hybride, se lient entre eux par covalence.

10. Procédé selon la revendication 9, **caractérisé en ce que** la liaison covalente comprend un traitement avec une ligase et/ou un comblement de lacunes dans les brins avec une ADN-polymérase.

11. Procédé selon l'une des revendications 6 à 10, **caractérisé en ce que** des séquences de reconnaissance pour l'interaction spécifique avec des molécules comme des protéines, des acides nucléiques, des peptides, des produits pharmaceutiques, des saccharides, des lipides, des hormones et/ou des composés organiques sont contenues dans la séquence à une ou plusieurs positions.

12. Procédé selon l'une des revendications 6 à 11, **caractérisé en ce que** la séquence de l'acide nucléique double brin est une séquence naturelle, une séquence non naturelle ou une combinaison des deux.

13. Procédé selon l'une des revendications 6 à 12, **caractérisé en ce que** la séquence est prise dans une banque de données, une expérience de séquençage ou un dispositif de synthèse intégrée et d'analyse de polymères.

14. Procédé selon l'une des revendications 1 à 13, **caractérisé en ce que** les blocs structuraux oligomères sont synthétisés par exposition séparée dans l'espace et/ou le temps à l'aide d'une matrice de sources de lumière programmable.

15. Procédé selon l'une des revendications 1 à 14, **caractérisé en ce que** l'on effectue une synthèse séparée dans l'espace et/ou le temps des blocs structuraux oligomères dans un support de réaction microfluidique ayant un ou plusieurs espaces de réaction fluidiques et une ou plusieurs zones de réaction à l'intérieur d'un espace de réaction fluidique.

16. Procédé selon l'une des revendications 1 à 15, **caractérisé en ce que** les éléments structuraux de la synthèse contiennent des nucléotides naturels, des nucléotides modifiés ou leurs mélanges.

17. Procédé selon l'une des revendications 1 à 16, **caractérisé en ce que** l'on utilise des éléments structuraux de synthèse modifiés pour le marquage, puis la détection des acides nucléiques double brin associés.

18. Procédé selon la revendication 17, **caractérisé en ce que** l'on utilise comme groupes de marquage des molécules à détecter en fonction de la lumière.

19. Procédé selon l'une des revendications 1 à 18 pour la préparation d'un acide nucléique double brin d'une séquence au choix en tant qu'agent thérapeutique ou pharmacologique.

20. Procédé selon l'une des revendications 1 à 18 pour la préparation d'un acide nucléique double brin d'une séquence au choix en tant qu'agent de diagnostic.

21. Procédé *in vitro* selon la revendication 19 ou 20, comprenant la délivrance directe de l'acide nucléique double brin préparé pour l'application envisagée.

22. Procédé *in vitro* selon la revendication 19 ou 20, comprenant une transformation dans des cellules effectrices.

23. Procédé selon l'une des revendications 1 à 18 pour la préparation d'un acide nucléique double brin d'une séquence au choix, dans lequel celui-ci est stabilisé, condensé et/ou topologiquement manipulé pendant ou après la combinaison et l'association par étapes.

24. Procédé selon la revendication 23, dans lequel la stabilisation, la condensation et/ou la manipulation topologique sont effectuées par des molécules fonctionnelles comme des histones ou des topoisomérases.

25. Procédé selon l'une des revendications 1 à 18 pour la préparation d'un acide nucléique double brin en tant que vecteur de clonage propageable, le vecteur de clonage propageable pouvant servir à la transcription, à l'expression de la séquence transcrite et éventuellement à la récupération de produits de gènes exprimés dans des cellules cibles appropriées.
